# EUROPEAN PATENT SPECIFICATION

(11) **EP 1 752 542 B1**
(45) Date of publication and mention of the grant of the patent: **27.05.2009**
(21) Application number: 05735152.0
(22) Date of filing: 04.04.2005
(51) Int. Cl.: C12N 15/89, A01K 67/027

(54) **METHOD OF GENERATING NON-HUMAN TRANSGENIC ANIMALS**
VERFAHREN ZUR ERZEUGUNG NICHTMENSCHLICHER TRANSGENER TIERE
PROCEDE PERMETTANT DE GENERER DES ANIMAUX TRANSGENIQUES NON HUMAINS

(30) Priority: 06.04.2004 ES 200400857
(43) Date of publication of application: 14.02.2007
(73) Proprietor: Consejo Superior de Investigaciones Cientificas, 28006 Madrid (ES); Instituto Nacional De Investigacion y Tecnologia agraria y Alimentaria (INIA), 28040 Madrid (ES)
(72) Inventor: VENTURA OLIVEIRA MOREIRA, Pedro Nuno, 28049 Madrid (ES); GUTIERREZ ADÁN, Alfonso, 28040 Madrid (ES); MOTOLIU JOSE , Lluís, CENTRO NAC. DE BIOTECNOLOGIA, 28049 MADRID (ES)
(74) Representative: Ungria Lopez, Javier
(86) International application number: PCT/ES2005/070038
(87) International publication number: WO 2005/098010

(56) References cited:
- WO-A1-00/09674
- MOREIRA P. ET AL.: 'Efficient generation of transgenic mice with intact yeast artificial chromosomes by intracytoplasmic sperm injection.' BIOL REPROD. vol. 71, no. 6, December 2004, pages 1943 - 1947, XP008073899
- PERRY A. ET AL.: 'Efficient metaphase II transgenesis with different transgene archetypes.' NAT BIOTECHNOL. vol. 19, no. 11, November 2001, pages 1071 - 1073, XP008073898
- MOREIRA P. ET AL.: 'Mouse ICSI with frozen-thawed sperm: the impact of sperm freezing procedure and sperm donor strain.' MOL REPROD DEV. vol. 66, no. 1, September 2003, pages 98 - 103, XP008073904
- AC P. ET AL.: 'Metaphase II transgenesis.' REPROD BIOMED ONLINE vol. 4, no. 3, May 2002, pages 279 - 284, XP008073903
- KUSAKABE H. ET AL.: 'Maintenance of genetic integrity in frozen and freeze-dried mouse spermatozoa.' PROC NATL ACAD SCI USA vol. 98, no. 24, November 2001, pages 13501 - 13506, XP002974775
- HIRABAYASHI M. ET AL.: 'Offspring derived from intracytoplasmic injection of transgenic rat sperm.' TRANSGENIC RES. vol. 11, no. 2, April 2002, pages 221 - 228, XP008073948
- SZCZYGIEL M. ET AL.: 'Expression of foreign DNA is associated with paternal chromosaome degradation in intracyplasmic sperm injection-mediated transgenesis in the mouse.' BIOL REPROD. vol. 68, no. 5, May 2003, pages 1903 - 1910, XP008073983
- SCHEDL A. ET AL.: 'A Yeast artificial chromosome covering the tyrosinase gene confers cpy number-dependent expression in transgenic mice.' NATURE vol. 362, no. 6417, 18 March 1993, pages 258 - 261, XP000563891

## Description

### SECTOR OF THE ART

The present invention provides a procedure for the production of non-human transgenic mammal animals by integrating large size DNA molecules (hundreds of kilobases) to be used in Biological, Biomedical and

Biotechnological applications. The relevance of this invention resides in the value of the transgenic animals that it generates, both in the sense of new products generated by these animals as for their use as experimental animals for the study or diagnosis of animal and human diseases, or even for their use in functional genomics to contribute, effectively, in the description of gene function.

### STATE OF THE ART

The production of transgenic animals by introducing exogenous DNA in the genome of their somatic or germ cells, provides us with experimental models to be used by the scientific community, but also with organisms for agricultural, biotechnological, pharmacological and medical applications. Some examples are the introduction of human genes in the mouse genome for the purpose of studying and diagnosing certain diseases; or the genetic modification of cows to produce in their milk, large quantities of proteins that are of interest to the pharmacological industry; or the functional study of new genes provided by the complete sequence of animal genomes.

Transgenic techniques are used mostly on animals used for laboratory experiments (mostly mice, but also rats and other rodents in less quantities) and in animals. The methodologies used to produce transgenic animals have evolved thanks to the development of new reproductive and molecular manipulation techniques. Amongst the most used techniques are the pronuclear microinjection of fertilized oocytes, the use of recombinant virus as mediators to introduce the transgenic gene in the embryonic genome, the use of embryonary pluripotent stem cells genetically altered by various methods, or dead or live sperm mediated DNA transfer (Nagy A., Gertstenstein M, Vintersten K, Behringer R. Manipulating the mouse embryo. A laboratory manual (3rd edition) Cold Spring Harbor Laboratory Press, Cold Spring Harbor. New York. 2003).

These methods have proved to be very effective when producing transgenic products with small DNA molecules, but there are no efficient and reproducible methods that allow the introduction of large DNA molecules (larger than 170 Kb) such as, for instance, yeast artificial chromosomes (YAC) or mammal artificial chromosomes (MAC) in the animal genome. As we learn the complexity of the animal genome, we realize we have to use longer DNA lengths in transgenic endeavors to be able to guarantee the joint introduction of all the coding genetic sequences and potentially regulating sequences, and in this manner ensure a correct gene expression, in time, place and level; and to study complex large genomic regions or structures that group various genes that work in a coordinated manner (i.e., the gene loci of immunoglobulins light or heavy chains) [Giraldo P., Montoliu L. (2201) Size matters: use of YACs, BACs and PACs in transgenic animals, Transgenic Research 10(2): 83 -103].

There are two methods used to produced transgenic organisms with artificial chromosomes: pronuclear microinjection of fertilized oocytes and manipulation of stem cells (transfected with DNA by lipofection, or by fusing the stem cells themselves with YAC-bearing yeast cells) [Peterson K.R. (2003), Transgenic mice carrying yeast artificial chromosomes. Expert Reviews in Molecular Biology 5: 1-25]. In what pertains to pronuclear microinjection, the internal diameter of the microinjection needle (less or equal to 1 micrometer) may prevent that large DNA molecules are introduced without breaking, as happens most times. When the size of the transgene is increased, the possibility of harming the DNA during purification, dilution, storage and mainly during manipulation is also increased. An alternative to microinjection is transforming the embryonary pluripotent stem cells with artificial chromosomes to later use these transformed cells in the production of chimeras by joining them with embryos in pre-implantational state, that can exceptionally transfer the transgene to their offspring. This protocol is very lengthy and costly, and that is why we propose an additional, faster and simpler protocol that will allow the efficient and reproducible introduction of large size sequences in the mammal genome. We have already demonstrated the efficacy of our protocol producing transgenic mice with YACs made of 250 kb DNA molecules, where the transgene has been introduced in a stable manner without breaks [Schedl A., Montoliu L., Kelsey G., Schütz G. (1993) A 250 kb YAC covering the tyrosinase gene confers position independent and copy number dependent expression in transgenic mice. Nature 362: 258 -261: Giraldo P. & Montoliu L. (2002) Artificial chromosome transgenesis in pigmentary research. Pigment Cell Research 15 (4) : 258 - 264], and we demonstrate for the first time that this sequence is transmitted in Mendelian fashion to the offspring, producing the expected phenotype.

Six patents related to the use of sperm to mediate the production of transgenic organisms have been found. These patents are:
- "Methods for producing transgenic animals" CA2394699 Schatten Gerald and Chan Anthony.
- "Methods of performing transgenesis" CA234042 Perry Anthony and Wakayama Teruhiko
- "Mammalian transgenesis by intracytoplasmatic sperm injection" CA2340199 Rapp Jeffrey and Sutrave Pramod
- "A method of transgenic embryo production comprising the coinsertion of a nucleic acid and a membrane-disrupted sperm head into the cytoplasm of unfertilized methaphase II oocyte". Tyuzo Yanagimachi.
- "The introduction and expression of large genomic sequences in transgenic animals" WO9423049. Gearhart John D; Lamb Bruce T.

Some of these patents mention the possible utilization of technology to create transgenic animals with large size DNA constructs, however, in none of the patents listed have these transgenic animals been made, nor can said transgenic animals be created using the technology described in said patents.

### DESCRIPTION OF THE INVENTION

### Brief description

The invention provides a method to obtain transgenic non-human mammal animals with large size DNA molecules (> 170 kb), preferably using yeast artificial chromosomes and mammal chromosomes. Briefly, this invention consists in generating transgenic embryos by co-microinjecting non-fertilized oocytes with sperm that has been previously joined to the transgenic DNA molecule. In this method we describe, for the first time, the specific fragmentation of both the membrane and sperm DNA, by a freeze-thawing process, in order to increase the efficiency of the integration of the transgene. Once the sperm have been subjected to thermal breakage treatment, they are co-incubated at 4° C with the artificial chromosome, that is then diluted in a specific buffer [Schedl A., Larin Z., Montoliu L., Thies E., Kelsey G., Lehrach H., Schütz G. (1993) A method for the generation of YAC transgenic mice by pronuclear microinjection. Nucleic Acids Research 21: 4783 - 4787; Montoliu L., Bock C.T., Schütz G. & Zentgraf H. (1995) Visualization of large DNA molecules by electron microscopy with polyamines: Application to the analysis of yeast endogenous and artificial chromosomes. Journal of Molecular Biology 246: 486 -493] that maintains the integrity of artificial chromosome DNA. The mixing process uses plastic tips with the ends cut off and is executed at low suction and expulsion speed to avoid fragmentation of the construct. After the co-incubation period (2 minutes) the sperm are injected in the oocyte.

The microinjection can be done by means of conventional mechanical micromanipulators, or more efficiently with a piezo-electric manipulator. The opening of the pipette used to intracytoplasmically inject the sperm must be greater than the diameter of the spermatozoon head and leave a gap between the sperm cell and the walls of the capillary vessel that allows circulation of large DNA molecules, avoiding in this manner that they break. In our invention we use microinjection pipettes that have a 7 µm internal diameter, a size that is 10 times larger than that of the usual pronuclear microinjection pipette, which enables the microinjection of very large sized DNA constructs without irreversible fragmentation.

The methodology of the invention is applicable to non-human mammals, but conceptually, to all animal species in which sperm and oocytes are involved in the fertilization process, not only vertebrates but also of invertebrates. With the methodology here described we have efficiently increased the integration of transgenic elements produced by sperm injection, and we have produced, for the first time, transgenic animals that can transmit a transgene (YAC) of 250 kb to their offspring, producing a phenotype that shows complete integration.

### Detailed description

The invention has the problem of providing new procedures to produce non-human transgenic mammal animals for exogenous DNA sequences or transgenes of interest, preferably large sized.

The solution provided by this invention is based on the inventors having shown that this new procedure is capable of producing transgenic animals bearing an yeast artificial chromosome (YAC) sized 250 kb, integrated in a stable manner in the genomic DNA, transmitted in a mendelian manner to their offspring and that it produces a phenotype (recovery of pigmentation) that indicates their whole presence (see Example 1), and in the case of using smaller exogenous DNA a greater frequency of transgenic animals has been obtained than when using other known methodologies (see Example 2). More specifically, with this procedure we have obtained a high percentage of transgenic animals (- 45%) using small DNA sequences (5 kb plasmids), a frequency that greatly exceeds that obtained with other methods; also, using 250 kb YACs we have obtained a high frequency of transgenic animals (YAC DNA bearers) of 35% and amongst them, we have detected animals that bear the DNA YAC molecule completely integrated in the genome of the host mouse with a ∼ 8 % frequency.

The main differences and advantages of our invention are:
1. Utilizing a new method of freezing that probably produces micro-breakages in the sperm DNA. The samples are for the first time kept at -80° C and for the first time in M2 media with no EDTA or EGTA, or decondensing elements, or restriction enzymes, as is the case with other procedures (Perry AC, Wakayama T, Kishikawa H., Kasai T, Okabe M, Toyoda Y, Yanagimachi R. 1999. Mammalian transgenesis by intracytoplasmic sperm injection. Science 284 /5417): 1180 -1183; Chan AW, Luetjens CM, Dominko T, Ramaho-Santos J, Simerly CR, Hewitson L, Schatten G. 2000. TransgenICSI reviewed: foreign DNA transmission by intracytoplasmic sperm injection in Rhesus monkey. Mol Reprod Dev 56 (2 Suppl): 325 - 328; Perry AC, Rothman A, de las Heras JI, Feinstein P, Mombaerts P, Cooke HJ, Wakayama T. 2001. Efficient metaphase II transgenesis with different transgene archetypes. Nat Biotechnol 19(11): 1071 -1073; Szczygiel MA, Moisyadi S, Ward WS. 2003. Expression of foreign DNA is associated with paternal chromosome degradation in intracytoplasmic sperm injection-mediated transgenesis in the mouse. Biol Reprod 6885): 1903 - 1910; Niemann H, Rath D, Wrenzycki C. 2003. Advances in biotechnology; new tools in future pig production for agriculture and biomedicine. Reprod Domest Anim 38 (2) :82-89). For the first time it is demonstrated that the breakage not only of the membrane but the probable breakage of the sperm DNA increases the efficiency of transgene integration.
2. Differences in co-incubation time between DNA and sperm. In our invention we use 2 min. instead of the 30 or 1 min. indicated in other procedures.
3. Use of large size yeast artificial chromosomes (not of small sequences, plasmids, BAC) to produce transgenic animals in combination with points 1 and 2. None of the known procedures have involved any experience with yeast artificial chromosomes. The scientific publication that most resembles this invention (although it does not employ YACs and the methodologies are different) is: Perry AC, Rothman A, de las Heras JI, Feinstein P. Mombaerts P, Cooke JH, Wakayama T. Efficient metaphase Ii transgenesis with different transgene archetypes, Nat Biotechnol. 2001 Nov;19(11): 1071 -3.
4. A new type of microinjection pipettes having a specific diameter that permits introducing large size DNA molecules without breaking them.
5. Our invention is the only system that selects the sperm that have suffered greater fragmentation (heads without tail post-defrosting) to increase transgenesis efficiency.

In short, the procedure herein described in this invention is the only one that has produced transgenic animals with yeast artificial chromosomes > 250 kb by injecting sperm, and has demonstrated for the first time that their integration is complete, stable, transmissible and produces the desired phenotype. Therefore, an object of the present invention is the procedure to generate transgenic animals, vertebrates and invertebrates, preferably vertebrates, and more preferably mammals that are not human, for exogenous DNA sequences or transgenes of variable dimensions, from now on transgenesis procedure of the invention, **characterized in that** they are generated from transgenic embryos by co-microinjection of sperm or sperm heads (in the case of mice), joined to said exogenous DNA sequence of interest in non-fertilized oocytes and because it is constituted by the following steps:
1) spermatic fragmentation by freeze-thawing of DNA and of the sperm or sperm head membranes and their keeping at - 80° C and in an isotonic buffered media without EDTA or EGTA,
2) production of the construct of DNA using as vectors for the transgene or for exogenous DNA sequence of interest, preferably large size sequences present in bacteriophages, cosmids, bacteria artificial chromosomes (BAC) or artificial chromosomes based on the replication origin of the P1 bacteriophage (PACs), and more preferably, yeast artificial chromosomes (YACs) or mammal artificial chromosomes (MACs),
3) mixture and incubation of DNA constructs with the sperm or defrosted-fragmented sperm heads during 2-5 minutes, preferably during 2 minutes,
4) micro-injection of constructs and sperm or defrosted-fragmented sperm heads into prepared phase II non-fertilized oocytes, using microinjection pipettes having a specific diameter that permits introducing large size DNA molecules, without breaking them, and, finally,
5) transfer of micromanipulated embryos to oviduct/uterus of a pseudo-pregnant receptive female to be able to continue development of transgenic progeny.

The term "exogenous DNA or transgene of interest" as used in the present invention refers to DNA that is normally not resident, nor present in more than one copy in the cell we intend to transform. Exogenous DNA may include, but not be limited to, DNA from mammals, plants, bacteria, viruses, bacteriophages, plasmids or synthetic constructs. The DNA may be a linear or circular molecule, with two chains, and may be inserted or part of the endogenous DNA, as referred by the term "exogenous DNA or transgene of interest" refers to said DNA sequence being capable of expressing in the transgenic animal one or more proteins of interest that permits, for instance, obtaining products of commercial interest generated by these animals (hormones, therapeutic factors, etc.), to use said animals as study models or to diagnose animal or human diseases, or to use them in functional genomics to contribute to the efficient description of genomic function [Bedell MA, Jenkins NA, Copeland NG. Mouse models of human disease. Part I: techniques in resources for genetic analysis in mice. Genes Dev. 1997 Jan 1; 1181): 1-10. Bedell Ma, Largaespada DA, Jenkins NA, Copeland NG. Mouse models of human disease. Part II: recent progress and future directions. Genes Dev. 1997 1:11(1)-43. Shashikant CS, Ruddle FH. 2003. Impact of transgenic technologies on functional genomics. Curr Issues Mol Biol 5(3): 75-98. Pintado B, Gutierrez-Adan A. 1999. Transgenesis in large domestic species: future development for milk modification. Reprod Nutr Dev 39(5-6):535-544.)
The term "exogenous DNA, variable size transgene of interest" as used in the present invention, refers to DNA of any size, at least over 5 kb, preferably a 170 kb, and more preferably equal or exceeding 250 kb.
The term "non-human mammals" as used in the present invention refers, amongst others, to mammals used as laboratory animals such as mice, rats and other rodents, and livestock animals such as cows, sheep, goats, pigs, rabbits and horses.

### 1.- Freeze-thawing - Spermatic fragmentation

A particular object of the present invention is the transgenesis procedure of the invention **characterized in that** spermatic fragmentation is done by a freezing-thawing thawing physical process in isotonic buffered media (preferably M2) in the absence of EDTA or EGTA, that despite being very aggressive, improves transgene integration (see Example 2), and that is done as follows:
a. The sperm or sperm head that is going to be frozen is uniformly re-suspended in a buffered isotonic media without EDTA or EGTA (preferably M2), until reaching a concentration of 1 to 3x10⁶ cells per milliliter,
b. 50 -100 µl Aliquots of the suspension are prepared in cryotubes (NUNC, Copenhagen), that are firmly closed and placed directly in liquid nitrogen (-196° C), during 10-15 minutes,
c. the aliquots are kept at -80° C (preventing thawing during the transition from -196° C to -80° C), and can be used during 3-4 weeks without embryo development being significantly impacted, and
d. finally, the sperm or sperm heads are defrosted at room temperature during 10 minutes and must then, be used for intracytoplasmic microinjection in non-fertilized oocytes in the next two hours, as described later [Moreira P.N., Jiménez A., Fernández R., Bury-Madrid N., De La Fuente J., Pintado B. y Gutiérrez-Adán A. (2003). Mouse ICSI with frozen-thawed sperm: The impact of sperm freezing procedure and sperm donor strain. Mol Reprod Dev 66 (1): 98 -103].

It must be noted that during placing of sperm cells in liquid nitrogen (b), it is very important to avoid contaminating the sample with liquid nitrogen, since it could compromise embryonary development and thus, full immersion of cryotubes must be avoided.

The methodology described can be applied to any sperm or sperm head sample (fresh, frozen, dehydrated, epidydimal or testicular) that maintain their fertilizing capacity and potential to generate normal embryonary development by microinjection in metaphase II non-fertilized oocytes. The methods to collect fresh sperm from freezing or dehydration, both from vertebrates as for invertebrates, are known by experts, and the freeze-thawing methodology that is presently described can be easily adapted/modified depending on the animal species or the type of sperm.

### 2.- Production of DNA constructs using yeast artificial chromosomes as vectors for the transgene

The procedures to make DNA constructs as vectors for the expression of sequences of interest are broadly known by experts in the field, and may be applicable for the present invention. For illustrational purposes, DNA of yeast artificial chromosomes (YAC) is obtained using methods previously described and optimized for their microinjection in mouse oocytes to generate transgenic mice (Schedl A., Grimes B. & Montoliu L. (1996) YAC-transfer by microinjection. In: Methods in Molecular Biology, volume 54, Yeast artificial chromosome protocols, ED: Markie D., chapter 25: 293 -306. Humana Press, Totowa (New Jersey); Lluis Montoliu. Large-scale preparation of agarose plugs of yeast AND (pag. 326-328). Purification of YAC AND with filtration units (pag. 329-331). Purification of YAC AND with filtration units (pag. 333). In: Manipulating the Mouse Embryo. A Laboratory Manual (Third Edition) (2003). Andras Nagy, Marian Gersenstein, Kristina Vintersten, Richard Behringer (Eds.), Cold Spring Harbor Laboratory Press. Cold Spring Harbor, New York) already applied to the generation of transgenic mice by means of pronuclear injection (Montoliu L., Umland T. & Schütz G. (1996) A locus control region at -12 kb of the tyrosinase gene. The EMBO Journal 15: 6026-6034).

Another particular object of the present invention is the transgenesis procedure of the invention in which the exogenous DNA or transgene of interest is found in a yeast artificial chromosome (YAC) that acts as vector. Another particular object of the present invention is the transgenesis procedure of the invention in which the transgene of interest is found in a mammal artificial chromosome (MAC) that acts as vector, such as for instance the human artificial chromosome (Larin Z., Mejia JE. Advances in human artificial chromosome technology. Trends Genet. 2002 Jun;18(6) : 313-9. Cooke H. Mammalian artificial chromosomes as vectors: progress and prospects. Cloning Stem Cells. 2001; 3(4):243-9. Hadlaczky G. Satellite ADN-Based artificial chromosomes for use in gene therapy. Curr Opin Mol Ther. 2001 Apr; 3(2): 125-32. Vos JM. Therapeutic mammalian artificial episomal chromosomes. Curr Opin Mol Ther. 1999 Apri; 1(2): 204-15).
Another particular object of the present invention is the transgenesis procedure of the invention in which the large size exogenous DNA or the transgene of interest is found in a plasmid, a bacteriophage, a cosmid, a BAC, a PAC, a YAC, a MAC or any other element that acts as a vector.

### 3.- Mix and incubation of yeast artificial chromosome bearers of the desired transgene with thawed sperm

The spermatic solution, thawed-fragmented and concentrated as described previously, is mixed with exogenous DNA contained in the yeast artificial chromosomes, also described previously. In this process, it is important to reduce the possibility of fragmentation of the constructs, as well as to maintain a specific final concentration of DNA (and associated macromolecules) present in the mixture, to avoid situations of toxicity for the embryo. The final DNA concentration may oscillate between 2 and 20 nanograms per microliter, preferably 10 nanograms for microliter of DNA-sperm solution. The concentration of DNA mother solution that contains the transgene, is prepared in such a manner that it can be diluted until reaching the desired DNA concentration, with a spermatic solution volume containing a number of spermatozoons (or heads) sufficiently elevated to not difficult the process of microinjection. For a 1:1 v:v dilution (DNA:spermatozoons) the minimum concentration of DNA must be found, ideally, around 10-20 µl in the mother solution.
Another particular object of the present invention is the transgenesis procedure **characterized in that** the mixing and incubation of sperm and DNA is done as follows:
1. initial samples are prepared in such a manner that final DNA concentration is equal to 10 nanograms per microliter of DNA-sperm solution,
2. samples are pipetted, in sterile conditions, using plastic tips with the heads cut off and slow suction and expulsion speed and at low temperature (4°C) to decrease the possibility of fragmentation of the constructs, and,
3. the DNA-sperm solution is incubated in ice during 2 minutes, before diluting it with 10% PVP in M2 (final DNA-sperm solution) to be microinjected at room temperature during the following 2 hours.

### 4.- Preparation of receptor oocytes

In the methodology of the invention, microinjections are done in mature oocytes that are in metaphase II, independently of the type of maturation (*in vivo* or *in vitro*) undergone. Immature oocytes (i.e, germinal vesicular stage) can be matured *in vitro* until they reach metaphase II state in maturation promoting media. The methodology for *in vitro* maturation of oocytes of diverse be found in Methods of Enzymology 225,77-84, Academic Press, 1993) and is accessible to any expert in the matter. Oocytes that have matured *in vivo* may be obtained from superovulated with injections of gonadotropin or other hormones, or by surgical means soon after ovulation. Oocytes that have been retrieved surgically from mice oviducts are enveloped in a cumulus cell mass that is dispersed by incubation in a buffered media containing testicular hyaluronidase (i.e., in mice, 300 IU/ml M2 during 3-5 minutes). Oocytes free of cumulus cells are then washed in a media without hyaluronidase and placed, until the moment of injection, in a culture media that has been previously balanced (i.e., KSOM + aminoacids for mice oocytes at 5% CO₂ and 37°C).

### 5.- Intracytoplasmic co-microinjection of sperm nucleus and exogenous DNA in the oocyte

During fertilization of oocytes in methaphase II by means of intracytoplasmic sperm microinjection (ICSI), the complete sperm may be injected. However, injecting the male gamete's tail must be avoided if its centrosome does not participate in the fertilization process (i.e., in mice). This facilitates the microinjection process and decreases the volume of fluid injected in the oocyte cytoplasm. Microinjection can be performed by an expert in the matter and conventionally [i.e., methodology for hamsters is described in Yanagida, K., Yanagimachi, R., Perrault, S. D. and R. G. Keinfield, Biology of Reproduction 44, 440-447 (1991); Perry AC, Wakayama T, Kishikawa H, Kasai T, Okabe M, Toyoda Y, Yanagimachi R. Mammalian transgenesis by intracytoplasmic sperm injection. Science. 1999 May 14; 284 (5417): 1180-3.], or helped by a piezo-electric microinjection unit (Piezo Impact Drive Unit from Prime Tech Ltd.; Tsukuda, Ibaraki-Ken, Japan). This unit uses the piezo-electric effect to move the injection pipette in a controlled back-and-forth fashion in short distances (0.5 micrometers approximately). The unit allows regulating the intensity and duration of each impulse.

For injection in the oocyte, the tail (if any) of a spermatozoon nucleus mixed with exogenous DNA is first drawn into the injection capillary. This capillary must have a tip (straight section provided a piezo-electric unit is used) with an internal diameter of about 6-10 µm (depending on the animal species) that allows introducing large size DNA molecules without breaking them. In some species, such as mouse, injecting the sperm head is sufficient for a normal embryonary development, and this simplifies the process of micromanipulation, as only heads that are separated during the freeze-thawing process -which are the heads that have probably suffered most during the freeze-thawing process - can be injected. Another particular object of the present invention is the transgenesis procedure **characterized in that** the process of co-microinjecting the constructs and sperm or frozen-thawed heads in prepared oocytes, is done, in the case of mice, previously selecting sperm heads that have lost their tail during the freeze-thawing process and in that the microinjection capillary has an internal diameter between approximately 6 and 10 µm, depending on the animal species, and preferably 7 µm.

During intracytoplasmic co-injection of the exogenous DNA-sperm complex, the oocyte is placed, by negative pressure and aided by a holding pipette, in such a manner that its metaphasic plate is placed furthest from the penetration point. The pellucid area is perforated by the application of piezo-electric impulses of sufficient intensity and velocity. After expelling the fragment of area that is adhered to the tip of the injection pipette, on the micromanipulation drop or in the perivitelline space of the oocyte, the sperm head approaches the opening of the injection pipette. The injection pipette injection pipette. The injection pipette is advanced to two thirds of the oocyte diameter, and at that point one sole minimal intensity impulse is applied to open the membrane. Penetration is indicated by the sagging of the oocyte's membrane. The exogenous DNA-sperm complex is released with a minimum fluid quantity (no more than 6 picoliters) into the oocyte's cytoplasm, thus transporting the transgene of interest. The injection pipette is then softly extracted from the oocyte cytoplasm to avoid the risk of cellular lysis. The microinjection is done as fast as possible in groups of 10-15 oocytes, which after 10-15 minutes of recovery in the microinjection drop are placed in culture conditions (i.e., KSOM media + aminoacids for mouse oocytes, balanced at 5% CO₂ and 37°C).

### 6.- Activation of fertilized oocytes

It is known that for oocyte activation to occur after microinjection without the aid of inductor chemical substances, it is necessary to somehow damage the sperm plasma membrane to permit release of internal components located perinuclearly (not completely characterized to date) into the oocyte cytoplasm. The action of these factors, already detectable at the round spermatid stage, is not species specific (i.e., porcine sperm factors have the capability to activate mice oocytes), and injection of one sole spermatozoon/head is sufficient to activate the recipient oocyte. In this invention, fragmentation of the sperm membrane is achieved by freeze-thawing, a method that permits the direct activation of the microinjected oocytes. In other species micromanipulation may be used to produce fragmentation of the sperm membrane, for example, immobilizing their tail against the bottom of the microinjection plate. The same is possible by treating the sperm with detergents such as SDS, Triton X-100, sonication or by freeze-dehydration. In some species (i.e., bovine species), the ICSI methodology used is not receiving oocytes. In those cases the normal methods used are: parthenogenetic activation methods such as electroactivation, injecting activating substances (i.e., adenophosphatin), or incubating oocytes in media that contains activating substances such as stimulators of internal Ca²⁺ release (caffeine, ionophore A 23187, ionomycin and ethanol), phosphoprotein signal modulators (2-aminopurin, staurosporin, sphingosine), inhibitors of protein synthesis (ciclohexamide, 6-dimethylaminopurin), or combinations thereof (ionomycin with 6-dimethylaminopurin).

### 7.- Development of embryos manipulated to produce offspring

Embryos that are developed *in vitro* until they reach the 2-8 cell, morule or blastocyte stage are transferred to the oviduct or womb of a pseudo-pregnant female. In mice, between 15-20 embryos are transferred for each receptor female.

Finally, the objective of the present invention is a transgenic animal, vertebrate or invertebrate, and more preferably a mammal that is not human, obtained by the transgenesis procedure described in the present invention. A non-human mammal, as referred, is preferably an animal placed in one of the following groups: laboratory animals such as mice, rats and other rodents, and livestock animals such as cows, sheep, goats, pigs, rabbits and horses.

### EXAMPLES OF THE EMBODIMENT

The following examples attempt to illustrate the methodology of the invention without limiting its scope.

### Example 1: production of transgenic mice that are bearers of a 250 kb yeast artificial chromosome by means of sperm injection

### Sperm Collection and Freeze-thawing

The epidydimal sperm was collected and uniformly suspended in M2 media (without EDTA or EGTA). The sperm cells thus collected were washed by centrifugation in a 1.5 ml polypropilene tube with 1 ml of fresh media and re-suspended to obtain a final concentration of 1-3 million spermatozoons per ml. Later 100 microliter aliquots of sperm solution were made in cryogenic tubes (NUNC, Copenhagen) that were correctly closed and placed directly in liquid nitrogen (-196°C) during 10-15 minutes, without being completely immersed in it to avoid liquid nitrogen contamination of the samples. Samples were later kept for periods of up to 4 weeks at -80° C (avoiding thawing during the transition from -196°C to - 80° C). Sterile conditions were maintained throughout the procedure.

Sperm samples were thawed at room temperature during 10 minutes before using them.

### Preparation of YAC DNA

DNA from yeast artificial chromosome (YAC) is obtained by methods that have already been described and optimized for being microinjected in mice oocytes to generate transgenic mice (Schedl A., Grimes B & Montoliu L. (1996) YAC-transfer by microinjection. In: methods in Molecular Biology, volume 54, Yeast artificial chromosome protocols, Ed: Markie D., chapter 25:294-306. Humana Press, Totowa (New Jersey); Lluis Montoliu. Large-scale preparation of agarose plugs of yeast ADN (pag. 326-328). Purification of YAC DNA with filtration units (pag. 329-331). Purification of YAC DNA with filtration units (pag. 333). In: Manipulating the Mouse Embryo. A Laboratory Manual (Third Edition) (2003). Andras Nagy, Marian Gersenstein, Kristina Vintersten, Richard Behringer (Eds.), Cold Spring Harbor Laboratory Press. Cold Spring Edition) (2003). Andras Nagy, Marian Gersenstein, Kristina Vintersten, Richard Behringer (Eds.), Cold Spring Harbor Laboratory Press. Cold Spring Harbor, New York) already applied to the generation of transgenic mice by means of pronuclear injection (Montoliu L., Umland T. & Schütz G. (1996) A locus control region at -12 kb of the tyrosinase gene. The EMBO Journal 15: 6026-6034). First, a saturating culture of the YAC of interest bearing yeast cells is obtained. After several concentrations and washes yeast cells are encapsulated in an agarose matrix with a low melting point shaped as little dice. These agarose dice are exposed to different enzymatic solutions and chemical treatments with detergents that lead to cell lysis and isolation of intact DNA molecules (including those corresponding to YACs). Separating the DNA molecule corresponding to the YAC from the remaining 16 chromosomes of the yeast cell is done by preparative electrophoresis in a pulsing field. Finally the DNA molecule corresponding to YAC is obtained in solution after having melted, under controlled conditions and in the presence of polyamines and a given ionic strength of media, the agarose by incubating it at 50° C and later digesting the liquated agarose. Centrifugal tubes coupled to dialysis membranes with a pore size that allows passage of all molecules smaller than 30,000 dalton are used to concentrate YAC DNA molecules. To purify the DNA concentrated solution of YAC, the solution is subjected to a 2-3 hours dialysis process at room temperature over floating filters with a 0.05 micrometer pore size against microinjection buffer (10mM Tris-HCl, pH = 7.5, 0.1 mM EDTA, 100 mM NaCl, 30 micromolar Espermina, 70 micromolar Espermidina). YAC DNA quantity is estimated by specific quantifying by fluorimetry and is verified by comparing fluorescence intensities in an agarose gel of horizontal electrophoresis against known quantities of YAC DNA. The utmost care and micropipette tips with the ends cut off to avoid breakage of YAC DNA molecules.

The YAC used was made in this manner, called YRT2ΔLCR, and has a size of 250 kb and includes the locus of mouse tyrosinase, a gene that codifies a fundamental enzyme in melanin synthesis. This YAC bears an internal deletion of a gene regulating area whose absence drastically reduces its expression in the skin, keeping its expression to the retina/eye, in a similar manner observed in other YACs previously described (Montoliu L., Umland T. & Schütz G. (1996) A locus control region at-12 kb of the tyrosinase gene. The EMBO Journal 15: 6026-6034; Gimenez E., Giraldo P., Jeffery G. & Montoliu L. (2001) Variegated expression and delayed retinal pigmentation during development in transgenic mice with a deletion in the locus control region of the tyrosinase gene. Genesis 30 (1): 21- 25; Giraldo P. & Montoliu L. (2002) Artificial chromosome transgenesis in pigmentary research. Pigment Cell Research 15 (4): 258-264).

### Preparation of the DNA-sperm mixture for the microinjector

The sperm solution, thaw-fragmented and concentrated as previously described is mixed with the yeast artificial chromosome DNA YRTΔLCR. The mixing process of the thaw-fragmented and concentrated sperm solution with DNA of yeast artificial chromosomes is done by pipetting, in sterile conditions, using plastic tips with the ends cut off, at a slow suction and expulsion speed and at low temperatures (4°C), to reduce the possibility of the constructs becoming fragmented. The final DNA concentration (and associated macromolecules) in the mixture is also important to avoid situations that are toxic for the embryo. Final DNA concentration must not exceed 10 nanograms per microliter of DNA-sperm solution. The concentration of DNA mother solution that contains toxic for the embryo. Final DNA concentration must not exceed 10 nanograms per microliter of DNA-sperm solution. The concentration of DNA mother solution that contains the transgene is prepared in such a manner that it can be diluted until reaching the desired final DNA concentration, with a sperm solution volume that contains a sufficiently high number of sperm (or heads) that does not hinder the microinjection process. For a 1:1 v:v dilution (DNA:sperm) the minimal DNA concentration must be, ideally, around 10-20 ng/µl in the mother solution. The concentration of the DNA mother solution that contained the transgene was 7.5 nanograms per microliter. The final DNA concentration in this experiment was of 3.75 nanograms per microliter (1:1 dilution of DNA:sperm). The DNA:sperm solution was cultured on ice during 2 minutes, before diluting it with 10% PVP in M2 to decrease adhesion to the injection pipette. This final DNA:sperm solution must be microinjected at room temperature during the following 2 hours.

### Intracytoplasmatic co-microinjection of sperm nucleus and exogenous DNA in the oocyte

During fertilization of metaphase II oocytes by intracytoplasmic injection of sperm (ICSI), the complete sperm may be injected. However, injecting the male gamete tail must be avoided if its centrosome does not participate in the fertilization process (i.e., in mice). This facilitates the microinjection process and decreases the volume of fluid injected in the oocyte cytoplasm. The microinjection can be performed in the conventional manner [i.e., methodology for hamsters is described in Yanagida, K., Yanagimachi, R., Perrault, S. D. and R. G. Keinfield, Biology of Reproduction 44, 440-447 (1991); Perry AC, Wakayama T, Kishikawa H, Kasai T, Okabe M, Toyoda Y, Yanagimachi R. Mammalian transgenesis by intracytoplasmic sperm injection. Science. 1999 May 14; 284 (5417): 1180-3.], or helped by a piezo-electric microinjection unit (Piezo Impact Drive Unit from Prime Tech Ltd.; Tsukuda, Ibaraki-Ken, Japan). This unit uses the piezo-electric effect to move the injection pipette in a controlled back-and-forth fashion in short distances (0.5 micrometers approximately). The unit allows regulating the intensity and duration of each impulse.

To inject a sperm nucleus mixed with exogenous DNA in the oocyte, the sperm tail (if any) is first drawn into the injection capillary. This capillary must have a tip (straight section provided a piezo-electric unit is used) with an internal diameter of about 6-10 µm (depending on the animal species). In some species, such as mouse, injecting the sperm head is sufficient for a normal embryonary development, and this simplifies the process of micromanipulation, and then only heads that are separated during the freeze-thawing process -which are the heads that have probably suffered the most during the freeze-fragmentation process - can be injected.

During intracytoplasmic co-injection of exogenous DNA-sperm complex, the oocyte is placed, by negative pressure and aided by a holding pipette, in such a manner that its metaphasic plate is placed furthest from the penetration point. The pellucid area is perforated by the application of piezo-electric impulses of sufficient intensity and speed. After expelling the fragment of area that is adhered to the tip of the injection pipette, on the micromanipulation drop or in the perivitelline space of the oocyte, the sperm head approaches the opening of the injection pipette. The injection pipette is advanced to two thirds of the oocyte diameter, and at that point an only minimal intensity impulse is applied to open the membrane. Penetration is indicated by sagging of the oocyte membrane. The exogenous DNA-sperm complex is released with a minimum fluid quantity (no more than 6 10-15 minutes of recovery in the microinjection drop are placed in culture conditions (i.e., KSOM media + aminoacids for mouse oocytes, balanced at 5% CO₂ and 37°C).

### Development of embryos manipulated to produce offspring

Embryos that are developed *in vitro* until they reach the 2-8 cell, morule or blastocyte stage are transferred to the oviduct or womb of a pseudo-pregnant female. In mice, between 15-20 embryos are transferred for each receptor animal.

### Genetic analysis of YAC integration in born mice

**Table 1. Embryonic division and transgenecity obtained with a 250 kb (YAC) construct**

| Technique used | Construct (size in kb) | Injected oocytes (n° sessions) | | Surviva 1 rate (%) | 2-cells (%) | Transferre d embryos (n° of recipients ) | | Live animals (%) | | Live transgen ic animals (%) | |
|---|---|---|---|---|---|---|---|---|---|---|---|
| IMPT | YRTΔLCR | 367 | (6) | 252 | ND¹ | 218 | (3) | 34 | (16) | 12 | (35) |
| | (250) | | | (69) | | | | | | | |

| | | | | | | | | | | | |
|---|---|---|---|---|---|---|---|---|---|---|---|
| IMPT (ICSI-mediated YAC Transfer); (1) Non-Determined | | | | | | | | | | | |

Additional Note: of the 12 live transgenic animals, at least 1 of them (8%) presents the totality of YAC YRTΔLCR sequence and manifests the expected phenotype (with pigmentation in the eyes and practically absent in the skin)

### Example 2. Use of a new protocol of freeze-thawing to increase efficiency of integration of transgenic elements produced by intracytoplasmatic injection of sperm

The effect of freezing the sperm in the presence or absence of chelating agents, that stabilize sperm chromosomes (EDTA), was analyzed in the transgene integration. In this example a plasmid bearer of GFP gene was used (a plasmid of approximately 5kb) under regulation of CMV promoter that produces a fluorescent protein easily detected in a fluorescence microscope. Using an intracytoplasmic microinjection protocol similar to that described in Example 1, the number of blastocytes that expressed the transgene were analyzed according to the freezing system used. The results indicated that when sperm was frozen with chelating agents the percentage of blastocytes expressing the transgene (GFP) was significantly lower (20 -30% of green blastocytes of 40 blastocytes analyzed) than when the freezing media did not contain said chelating agents (100% of green blastocytes of 40 blastocytes analyzed). Later, this freeze-fragmentation protocol was used to produce transgenic mice and 45% of transgenic animals were obtained, a frequency that greatly exceeds that obtained with other methods (Table 2) and in which the transgene was incorporated integrally in all cases.

**Table 2. Embryonic division and transgenecity obtained with a 5.3kb (EGFP) construct**

| Technique used | Construct (size in kb) | | Injected oocytes (n° sessions) | | Survival rate (%) | 2-cells (%) | Transferred embryos (n° of recipients ) | | Live animals (%) | Live transgenic animals (%) | |
|---|---|---|---|---|---|---|---|---|---|---|---|
| IMPT | EGFP | (5.4 ) | 269 | (6) | 195 | 167 | 163 | (8) | 22 (13) | 10 | (45) |
| | | | | | (78) | (86) | | | | | |

| | | | | | | | | | | | |
|---|---|---|---|---|---|---|---|---|---|---|---|
| IMPT (ICSI-Mediated Plasmid Transfer) | | | | | | | | | | | |

### MATERIALS AND METHODS

### Laboratory Animals

CD-1 mice (6-8 week-old females and 3-8 month-old males) were used as oocytes and sperm donors. Female mice receptors for the embryos were CD-1 females previously crossed with vasectomized mice of the same strain to induce pseudo-pregnancy. Animals were fed *ad libitum* with a conventional diet and kept in a room with controlled temperature and light (23 °C, 14 hours of light; 10 hours of darkness). All experiments involving animals were performed according to the guidelines specified in the Guide for the Care and Use of the European Federation of Laboratory Animal Science Associations.

### Oocyte Collection and Preparation

Oocytes in metaphase II (MII) were collected 14 hours after administering human chorionic gonadotropin (HCG), obtained from females superovulated with 5 IU of PMSG and an equivalent dose, administered 48 hours later of HCG. The cumulus cells were dispersed by 3-5 minutes incubation in M2 media that contained 350 IU/ml of hyaluronidase.

The oocytes free of cumulus cells were then washed in a hyaluronidase free media and placed, in a previously balanced culture media until the moment of microinjection (i.e. KSOM + aminoacids for mice oocytes at 5% CO₂ and 37° C).

### Embryonary Micromanipulation, Culture and Transfer

Pronuclear injection in mice has been done in the conventional manner (Nagy A., Gertstenstein M, Vintersten K, Behringer R. Manipulating the mouse embryo. A laboratory manual (3rd edition) Cold Spring Harbor Laboratory Press, Cold Spring Harbor. New York. 2003). The ICSI-Assisted YAC Integration performed with freeze-thawed sperm has been done in M2 media, at room temperature, immediately after mixing sperm and DNA, during a period not exceeding 120 minutes. A volume of sperm-YAC solution has been mixed with 5 of M2 media containing 10% polyvynil-pyrrolidone (PVP) to decrease adhesion. The ICSI plate contained a manipulation drop (M2 media), a sperm-YAC drop (sperm-YAC solution in M2/10% PVP) and a drop of M2/10% PVP to clean the injection needle. Injections have been performed with a piezo-electric unit using a mercury-containing pipette, with a straight tip and internal diameter of 5-6 micrometers. Individualized heads obtained after freeze-thawing were co-injected with DNA into the oocytes. Oocytes were injected in groups of 10. After 15 minutes of recovery at room temperature in M2 media, surviving oocytes were placed in KSOM covered with mineral oil and cultured at 37° C in a 5% CO₂ atmosphere. To obtain offspring, embryos pronuclearly injected or fertilized by ICSI were transferred to the oviducts of recipient CD-1 pseudo-pregnant female recipients.

## Claims

1. Process for the generation of non-human transgenic animals for exogenous DNA sequences or transgenes, generated from transgenic embryos by co-microinjection of sperm or sperm heads, **characterized in that** it comprises the following steps:
- fragmentation of both the membrane and sperm DNA by a freeze-thawing process in a buffered isotonic medium that does not contain EDTA or EGTA,
- production of a DNA construct using a vector for the transgene or exogenous DNA sequence of interest, of a size larger than 170 kb,
- mixture and incubation of said DNA construct with said thaw-fragmented sperm during a time of 2 to 5 min,
- co-microinjection of said DNA construct and thaw-fragmented sperm into non-fertilized metaphase II prepared oocytes, and
- transfer of the resulting micromanipulated embryos to the oviduct/womb of a pseudo-pregnant receptor female to enable the ensuing development of transgenic offspring.

2. Process according to claim 1, **characterized in that** said animals are vertebrate animals.

3. Process according to claim 2, **characterized in that** said vertebrate animals are mammals.

4. Process according to claim 3, **characterized in that** said mammals are rodents.

5. Process according to claim 3, **characterized in that** said mammals are livestock animals.

6. Process according to any of claims 1 to 5, **characterized in that** said buffered isotonic media is M2.

7. Process according to any of claims 1 to 6, **characterized in that** said DNA construct is of a size of 250 kb or larger.

8. Process according to any of claims 1 to 7, **characterized in that** said vector is a bacteriophage.

9. Process according to any of claims 1 to 7, **characterized in that** said vector is a cosmid.

10. Process according to any of claims 1 to 7, **characterized in that** said vector is a bacterial artificial chromosome, BAC.

11. Process according to any of claims 1 to 7, **characterized in that** said vector is an artificial chromosome based on the replicaton origin of the P1 bacteriophage, PAC.

12. Process according to any of claims 1 to 7, **characterized in that** said vector is a yeast artificial chromosome, YAC.

13. Process according to any of claims 1 to 7, **characterized in that** said vector is a mammal artificial chromosome, MAC.

14. Process according to any of claims 1 to 13, **characterized in that** said incubation takes 2 min of time.

15. Process according to any of the preceding claims, **characterized in that** it comprises the following steps:
- fragmentation of both the membrane and sperm DNA by a freeze-thawing process in a buffered isotonic medium that does not contain EDTA or EGTA,
- production of a DNA construct of 250 kb or larger using as vector for the transgene or exogenous DNA sequence of interest a bacteriophage, a cosmid, a BAC, a PAC, a YAC or a MAC,
- mixture and incubation of said DNA construct with said thaw-fragmented sperm during 2 min of time,
- co-microinjection of said construct and thaw-fragmented sperm into non-fertilized metaphase II prepared oocytes, and
- transfer of the resulting micromanipulated embryos to the oviduct/womb of a pseudo-pregnant receptor female to enable the ensuing development of transgenic offspring.

## Patentansprüche

1. Verfahren zur Herstellung von nicht-menschlichen, transgenen Tieren für exogene DNA-Sequenzen oder Transgene, die aus transgenen Embryos durch Co-Mikroinjektion von Sperma oder Spermaköpfen produziert wurden, **dadurch gekennzeichnet, dass** es die folgenden Schritte umfasst:
- Fragmentieren sowohl der Membranen- als auch der Sperma-DNA durch einen Gefrier-Auftau-Prozess in einem gepufferten isotonischen Medium, das kein EDTA oder EGTA enthält;
- Herstellen eines DNA-Konstrukts unter Verwendung eines Vektors für die jeweilige transgene oder exogene DNA-Sequenz, mit einer Größe über 170 kb;
- Mischen und Inkubieren des DNA-Konstrukts mit dem taufragmentierten Sperma in einer Zeit von 2 bis 5 min;
- Co-Mikroinjizieren des DNA-Konstrukts und des tau-fragmentierten Spermas in nicht-fertilisierte, in der Metaphase II hergestellte Oozyten; und
- Transferieren des resultierenden mikromanipulierten Embryos zum Eileiter/zur Gebärmutter eines pseudo-schwangeren Rezeptorweibchens, um die Entwicklung eines transgenen Nachkommen sicherstellen zu können.

2. Verfahren nach Anspruch 1, **dadurch gekennzeichnet, dass** die Tiere Wirbeltiere sind.

3. Verfahren nach Anspruch 2, **dadurch gekennzeichnet, dass** die Wirbeltiere Säugetiere sind.

4. Verfahren nach Anspruch 3, **dadurch gekennzeichnet, dass** die Säugetiere Nagetiere sind.

5. Verfahren nach Anspruch 3, **dadurch gekennzeichnet, dass** die Säugetiere Vieh sind.

6. Verfahren nach einem der Ansprüche 1 bis 5, **dadurch gekennzeichnet, dass** das gepufferte isotonische Medium M2 ist.

7. Verfahren nach einem der Ansprüche 1 bis 6, **dadurch gekennzeichnet, dass** das DNA-Konstrukt eine Größe von 250 kb oder mehr aufweist.

8. Verfahren nach einem der Ansprüche 1 bis 7, **dadurch gekennzeichnet, dass** der Vektor ein Bakteriophage ist.

9. Verfahren nach einem der Ansprüche 1 bis 7, **dadurch gekennzeichnet, dass** der Vektor ein Cosmid ist.

10. Verfahren nach einem der Ansprüche 1 bis 7, **dadurch gekennzeichnet, dass** der Vektor ein bakterielles künstliches Chromosom (BAC, "bacterial artificial chromosome") ist.

11. Verfahren nach einem der Ansprüche 1 bis 7, **dadurch gekennzeichnet, dass** der Vektor ein künstliches Chromosom ist, das auf dem Replikationsursprung des P1-Bakteriophagen basiert (PAC, "P1-artificial chromosome").

12. Verfahren nach einem der Ansprüche 1 bis 7, **dadurch gekennzeichnet, dass** der Vektor ein künstliches Hefechromosom (YAC, "yeast artificial chromosome") ist.

13. Verfahren nach einem der Ansprüche 1 bis 7, **dadurch gekennzeichnet, dass** der Vektor ein künstliches Säugetierchromosom (MAC, "mammal artificial chromosome") ist.

14. Verfahren nach einem der Ansprüche 1 bis 13, **dadurch gekennzeichnet, dass** das Inkubieren 2 min dauert.

15. Verfahren nach einem der vorhergehenden Ansprüche, **dadurch gekennzeichnet, dass** es weiters die folgenden Schritte umfasst:
- Fragmentieren sowohl der Membranen- als auch der Sperma-DNA durch einen Gefrier-Auftau-Prozess in einem gepufferten isotonischen Medium, das kein ETDA oder EGTA enthält;
- Herstellen eines DNA-Konstrukts von 250 kb oder mehr, unter Verwendung eines Bakteriophagen, eines Cosmids, eines BAC, eines PAC, eines YAC oder eines MAC als Vektor für die jeweilige transgene oder exogene DNA-Sequenz;
- Mischen und Inkubieren des DNA-Konstrukts mit dem taufragmentierten Sperma in einer Zeit von 2 min;
- Co-Mikroinjizieren des Konstrukts und des tau-fragmentierten Spermas in nicht-fertilisierte, in der Metaphase II hergestellte Oozyten; und
- Transferieren des resultierenden mikromanipulierten Embryos zum Eileiter/zur Gebärmutter eines pseudo-schwangeren Rezeptorweibchens, um die Entwicklung eines transgenen Nachkommen sicherstellen zu können.

## Revendications

1. Procédé pour la génération d'animaux transgéniques non humains pour des séquences d'ADN exogène ou des transgènes, générés à partir d'embryons transgéniques par co-microinjection de spermatozoïdes ou de têtes de spermatozoïde, **caractérisé en ce qu'**il comprend les étapes suivantes :
- fragmentation à la fois de l'ADN de membrane et de spermatozoïde par un procédé de congélation-décongélation dans un milieu isotonique tamponné qui ne contient pas d'EDTA ni d'EGTA,
- production d'une construction d'ADN en utilisant un vecteur pour le transgène ou la séquence d'ADN exogène d'intérêt, d'une taille supérieure à 170 kb,
- mélange et incubation de ladite construction d'ADN avec ledit spermatozoïde décongelé fragmenté durant un temps de 2 à 5 minutes,
- co-microinjection de ladite construction d'ADN et du spermatozoïde décongelé fragmenté dans des ovocytes en métaphase II préparés non fertilisés, et
- transfert des embryons micromanipulés résultants à l'oviducte/abdomen d'un receveur femelle pseudo-gravide pour permettre le développement ultérieur de la descendance transgénique.

2. Procédé selon la revendication 1, **caractérisé en ce que** lesdits animaux sont des animaux vertébrés.

3. Procédé selon la revendication 2, **caractérisé en ce que** lesdits animaux vertébrés sont des mammifères.

4. Procédé selon la revendication 3, **caractérisé en ce que** lesdits mammifères sont des rongeurs.

5. Procédé selon la revendication 3, **caractérisé en ce que** lesdits mammifères sont des animaux d'élevage.

6. Procédé selon l'une quelconque des revendications 1 à 5, **caractérisé en ce que** ledit milieu isotonique tamponné est M2.

7. Procédé selon l'une quelconque des revendications 1 à 6, **caractérisé en ce que** ladite construction d'ADN est d'une taille de 250 kb ou plus.

8. Procédé selon l'une quelconque des revendications 1 à 7, **caractérisé en ce que** ledit vecteur est un bactériophage.

9. Procédé selon l'une quelconque des revendications 1 à 7, **caractérisé en ce que** ledit vecteur est un cosmide.

10. Procédé selon l'une quelconque des revendications 1 à 7, **caractérisé en ce que** ledit vecteur est un chromosome artificiel de bactérie, BAC.

11. Procédé selon l'une quelconque des revendications 1 à 7, **caractérisé en ce que** ledit vecteur est un chromosome artificiel basé sur l'origine de réplication du bactériophage Pl, PAC.

12. Procédé selon l'une quelconque des revendications 1 à 7, **caractérisé en ce que** ledit vecteur est un chromosome artificiel de levure, YAC.

13. Procédé selon l'une quelconque des revendications 1 à 7, **caractérisé en ce que** ledit vecteur est un chromosome artificiel de mammifère, MAC.

14. Procédé selon l'une quelconque des revendications 1 à 13, **caractérisé en ce que** ladite incubation prend 2 minutes de temps.

15. Procédé selon l'une quelconque des revendications précédentes, **caractérisé en ce qu'**il comprend les étapes suivantes :
- fragmentation à la fois de l'ADN de membrane et de spermatozoïde par un procédé de congélation-décongélation dans un milieu isotonique tamponné qui ne contient pas d'EDTA ni d'EGTA,
- production d'une construction d'ADN de 250 kb ou plus, en utilisant comme un vecteur pour le transgène ou la séquence d'ADN exogène d'intérêt, un bactériophage, un cosmide, un BAC, un PAC, un YAC ou un MAC,
- mélange et incubation de ladite construction d'ADN avec ledit spermatozoïde décongelé fragmenté durant un temps de 2 minutes,
- co-microinjection de ladite construction d'ADN et du spermatozoïde décongelé fragmenté dans des ovocytes en métaphase II préparés non fertilisés, et
- transfert des embryons micromanipulés résultants à l'oviducte/abdomen d'un receveur femelle pseudo-gravide pour permettre le développement ultérieur de la descendance transgénique.
